# EUROPEAN PATENT SPECIFICATION

(11) **EP 1 932 466 B1**
(45) Date of publication and mention of the grant of the patent: **08.06.2011**
(21) Application number: 07023173.3
(22) Date of filing: 29.11.2007
(51) Int. Cl.: A61B 5/022

(54) **Blood pressure measuring device**
Blutdruckmessgerät
Dispositif de mesure de la pression artérielle

(30) Priority: 14.12.2006 JP 2006337376
(43) Date of publication of application: 18.06.2008
(73) Proprietor: Panasonic Electric Works Co., Ltd., Kadoma-shi Osaka (JP)
(72) Inventor: Kojima, Takeshi, Kadoma-shi Osaka 571-8686 (JP); Yuasa, Tsuyoshi, Kadoma-shi Osaka 571-8686 (JP); Fumuro, Shinichi, Kadoma-shi Osaka 571-8686 (JP); Kanetsuna, Yoshitoshi, Kadoma-shi Osaka 571-8686 (JP)
(74) Representative: Appelt, Christian W.

(56) References cited:
- EP-A- 0 542 413
- WO-A-00/10628
- WO-A-2005/083940
- WO-A1-2006/039920
- JP-A- 2006 204 543
- US-A- 5 083 457
- US-A1- 2003 002 685
- US-A1- 2004 018 817
- US-B1- 6 344 025

## Description

### TECHNICAL FIELD

The present invention relates to a blood pressure measuring device which measures a blood pressure of a user in a state where a cuff is mounted on a part to be measured.

### BACKGROUND ART

A blood pressure measuring device for measuring a blood pressure of a user in a state where a cuff is wrapped around e.g. an upper arm of the user has been disclosed. Japanese Non-examined Patent Publication No. 2006-204543 discloses a blood pressure measuring device which comprises a main device for measuring a blood pressure of a user by using a cuff, and a separate device which can be used separately from the main device. The main device includes a pressurizing means for inflating a cuff to constrict a blood flow, and a control means for controlling the pressurizing means to adjust a pressure of the cuff and measure the blood pressure, and a wireless communication means. The separate device includes a wireless communication means for performing a wireless communication with the wireless communication means of the main device, a display for displaying data received from the main device via the wireless communication means, and an operating part for operating the main device via the second wireless communication means. In this blood pressure measuring device, it is possible for the user to check the measured result measured by the main device on the display of the separate device.

In such a blood pressure measuring device having the main device and the separate device, the blood pressure measuring device consumes energy for the wireless communication between the main device and the separate device. In a case where a power supply source for supplying power to each of the main device and the separate device is a battery, if the power consumption used for the wireless communication is large, the number of times that the blood pressure measuring device can be used may be reduced. Furthermore, it is desired that the main device and the separate device are reduced in size and weight as much as possible, so it is difficult to increase the size of the power supply source. Especially, it is difficult to increase the size of the separate device because, if the size of the separate device is increased, it becomes difficult to carry the separate device. Therefore, it is necessary to reduce the power consumption of the wireless communication between the main device and the separate device.

From EP 0 542 413 A1 an ambulatory patient monitoring system is known showing the features of the preamble of claim 1. From US 6,344,025 B1, JP 2006-204543 A, WO 00/10628, WO 2005/083940 A1 and from US 2003/0002685 A1 additional devices are known showing general technological background for the present invention.

From WO 2006/039920 A1 a communication device control device and communication system are known. The disclosed communication device is able to carry out process steps which bring the same alternately into an activated or deactivated state.

Moreover, from US 5,083,457 a remotely actuated tire pressure sensor and from US 2004/0018817 A1 a communication system and communication control method are known.

### DISCLOSURE OF THE INVENTION

In view of the above problem, the object of the present invention is to provide a blood pressure measuring device comprising a main device and a separate device, which can constrain power consumption necessary for the wireless communication between the main device and the separate device, and can reduce the size and the weight of the blood pressure measuring device.

This object is solved by a blood pressure measuring device according to claim 1 or claim 2.

The blood pressure measuring device in accordance with the present invention comprises a main device, and a separate device capable of being used separately from said main device. The main device has a pressurizing means for inflating a cuff, a control means for controlling the pressurizing means to measure a blood pressure of a user, and a wireless communication means. The separate device has a wireless communication means for performing a wireless communication with the wireless communication means of the main device, and at least one of a display means for displaying data received from the main device via the wireless communication and an operation means for operating the main device via the wireless communication. At least one device of the main device and the separate device has a power switch, and one device having the power switch is activated when the power switch is operated when the one device is in a power-off state, and then the one device transmits a start signal to the other device by the wireless communication. The other device shifts periodically from a power-off state to a power-on state on its own when it is in the power-off state in order to check the start signal, and it is activated if it receives the start signal when it shifts from the power-off state to the power-on state, and it returns to the power-off state if it does not receive the start signal when it shifts from the power-off state to the power-on state. The one device transmits the start signal continuously over a period longer than a period during which the power switch is operated to activate the one device.

If the start signal is transmitted only a period over which the power switch is operated by a user to activate the one device, the other device have to shift from the power-off state to the power-on state in a very short cycle when it is in the power-off state in order to check the start signal. So, in that case, the other device consumes a large amount of current.

In the present invention, because the start signal is transmitted continuously over a period longer than a period during which the power switch is operated by a user to activate the device, so it is possible to set the cycle, in which the other device shifts from the power-off state to the power-on state to check the start signal, long. Therefore, it is possible to constrain the power consumption necessary for the wireless communication between the main device and the separate device, and to reduce the size and the weight of the blood pressure measuring device.

The one device starts to transmit the start signal at the same time when the power switch is operated by the user to activate the device, and it continuously transmits the start signal over a period during which the power switch is operated, and it continuously transmits the start signal over a predetermined period after completion of the operation of the power switch.

Or, the other device may transmit an acknowledgment signal when it receives the start signal, and the one device may start to transmit the start signal at the same time when the power switch is operated to activate the device, and the one device may stop transmitting the start signal when it receives the acknowledgment signal from the other device.

### BRIEF DESCRIPTION OF THE DRAWINGS

FIG. 1 is an external view of a blood pressure measuring device in accordance with a first embodiment of the present invention.
FIG. 2 is a block diagram of the blood pressure measuring device of FIG. 1.
FIG. 3A is a flow chart for explaining an operation for blood pressure measurement of the blood pressure measuring device of FIG. 1.
FIG. 3B is a flow chart for explaining an operation of a separate device of the blood pressure measuring device of FIG. 1.
FIG. 4 is a timing chart on startup of the blood pressure measuring device of FIG. 1.
FIG. 5 is a timing chart on startup of the blood pressure measuring device in accordance with a second embodiment of the present invention.
FIG. 6A is an external view of a blood pressure measuring device in accordance with a third embodiment of the present invention.
FIG. 6B is an external view of a blood pressure measuring device in accordance with a fourth embodiment of the present invention.
FIG. 6C is an external view of a blood pressure measuring device in accordance with a fifth embodiment of the present invention.
FIG. 7A to 7C are views for explaining a blood pressure measuring device.
FIG. 8A to 8D are views for explaining modified forms of the separate device of FIG. 7A.
FIG. 9 is a view for explaining another modified form of the separate device of FIG. 7A.

### BEST MODE FOR CARRYING OUT THE INVENTION

Hereinafter, the present invention will be described in more detail with reference to the accompanying drawings.

### (First embodiment)

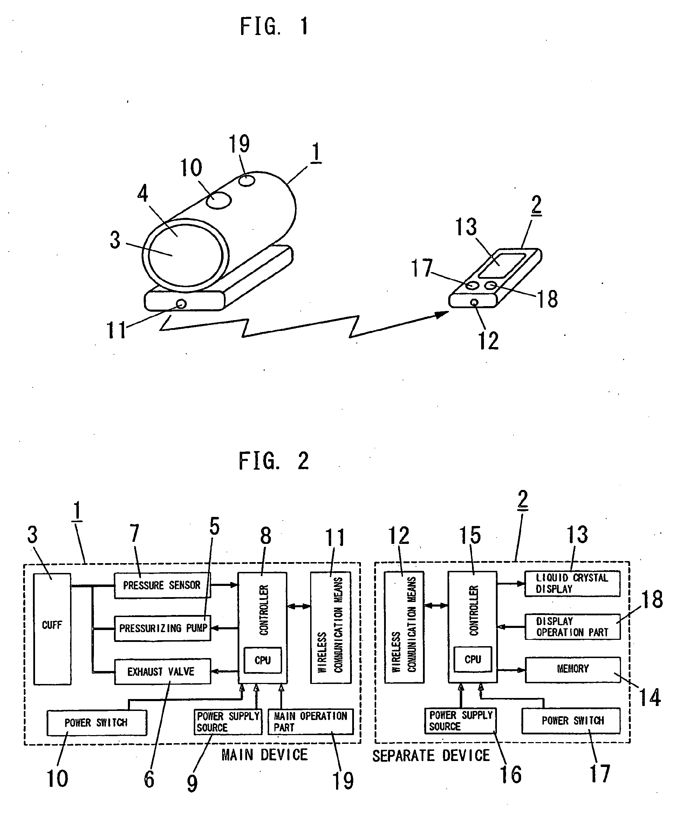

As shown in FIG. 1 and FIG. 2, the blood pressure measuring device in accordance with a first embodiment of the present invention has a main device 1, and a separate device 2 which can be used separately from the main device 1. As described in detail later, the separate device 2 of this embodiment is configured to be turned on in conjunction with the main device 1.

The main device 1 includes a through hole 4 though which an arm of a user is inserted. Furthermore, the main device 1 includes a cuff 3 which is disposed along an inner surface of the through hole 4 and is for constricting a blood flow, a pressurizing pump 5 (a pressurizing means) for inflating (pressurizing) the cuff 3, an exhaust valve 6 for exhausting air from the cuff 3, a pressure sensor 7 for detecting a pressure of the cuff 3, a main operation part 19 for providing instructions to the main device 1 from outside to perform a blood pressure measurement, and a controller 8 for controlling the pressurizing pump 5 and the exhaust valve 6 according to the instructions from the main operation part 19 and the pressure value of the cuff 3 detected by the pressure sensor 7 to adjust the pressure of the cuff 3 and measure the blood pressure. The controller 8 includes CPU for calculating a blood pressure and so on. A power supply source 9 for supplying power to the main device 1, a power switch 10 for turning on/off the main device 1, and a wireless communication means 11 are connected to the controller 8. In this embodiment, the power supply source 9 consists of storage batteries or dry batteries, and so on. The cuff 3 may be provided separately from the main device 1. For example, the cuff 3 may be connected to the main device 1 through a pipe, a tube, and so on.

The separate device 2 has a wireless communication means 12 for performing a wireless communication with the wireless communication means 11 of the main device 1, a liquid crystal display (display means) 13 for displaying various information, a nonvolatile memory 14 for storing data, and a controller 15 for displaying various measured data, e.g. a pressure value, a blood pressure value, a pulse rate, and so on, received from the main device 1 via the wireless communication on the liquid crystal display 13 and for storing the data in the nonvolatile memory 14. Furthermore, a power supply source 16 for supplying power to the separate device 2, a power switch 17 for turning on/off the separate device 2, and a display operation part 18 for switching the content displayed on the liquid crystal display 13 are connected to the controller 15. In this embodiment, the power supply source 16 consists of storage batteries or dry batteries, and so on.

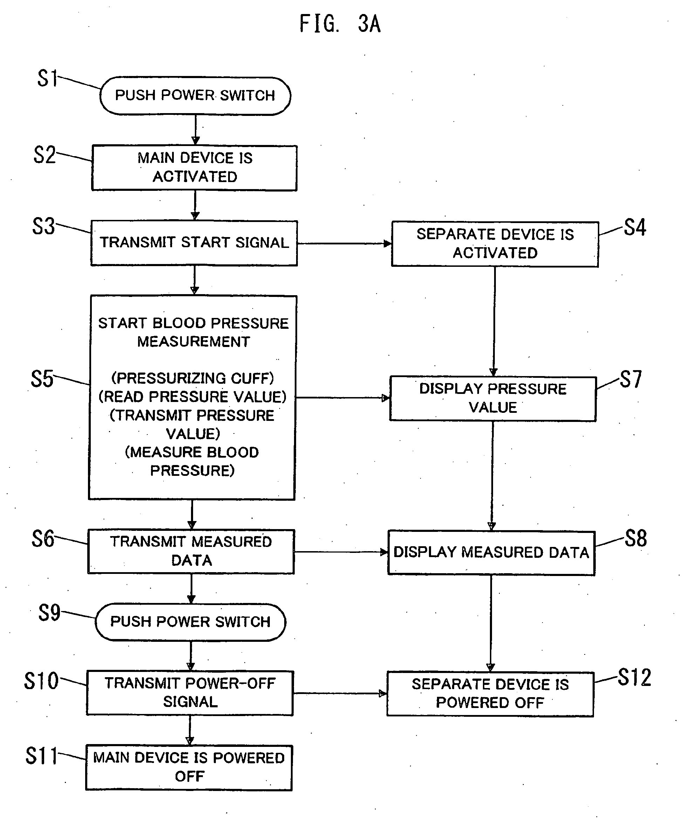

When a user measures the blood pressure by using this blood pressure measuring device, the user puts his/her arm through the through hole 4 of the main device 1, and he or she holds his/her posture in a state where his/her upper arm faces the cuff 3. Then, as shown in FIG. 3A, the user pushes the power switch 10 to activate the main device 1 (step S1 and S2). Upon activation of the main device 1, a start signal is transmitted from the wireless communication means 11 of the main device 1 (step S3), and the separate device 2 is also activated when it receives the start signal (step S4). The method of the wireless communication between the main device 1 and the separate device 2 on startup will be described in detail later. When the main device 1 and the separate device 2 are both activated, a blood pressure measurement is started (step S5). That is, the controller 8 controls the pressurizing pump 5 to pressurize (inflate) the cuff 3, and measures the blood pressure of the user. During the measurement, the controller 8 reads the pressure value of the cuff 3 detected by the pressure sensor 7, and transmits it to the separate device 2. And, the measured result, such as a blood pressure value and a pulse rate, obtained by the main device 1 is also transmitted from the wireless communication means 11 of the main device 1 to the wireless communication means 12 of the separate device 2 by wireless communication (step S6). The separate device 2 displays the received data, such as the pressure value of the cuff 3, the blood pressure value, and the pulse rate, on the liquid crystal display 13 (step S7 and S8), and stores necessary data in the memory 14. After the completion of the measurement, when the user pushes the power switch 10 of the main device 1 again (step S9), a power-off signal is transmitted from the wireless communication means 11 of the main device 1 (step S10), and the main device 1 is turned off (step S11). When the separate device 2 receives the power-off signal, the separate device 2 is also turned off (step S12).

Although, in this embodiment, the blood pressure measurement is automatically started when the power switch 10 is pushed, the blood pressure measurement may be started when a user operates the main operation part 19, not in conjunction with the power switch 10.

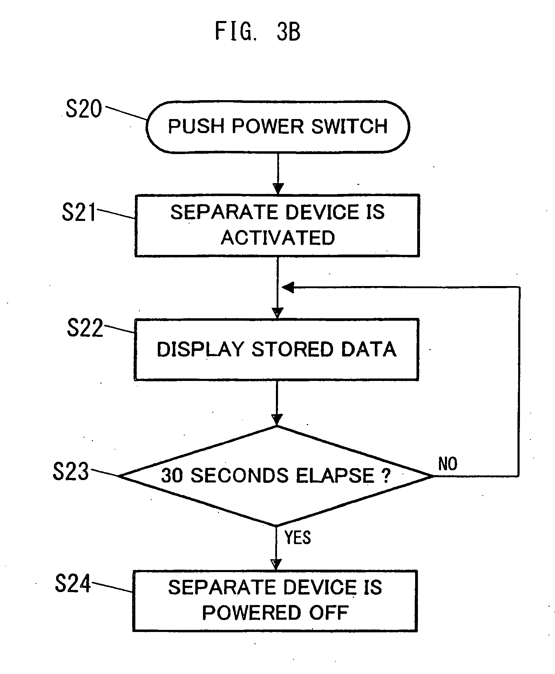

Because, in the blood pressure measuring device of this embodiment, the liquid crystal display 13 is provided in the separate device 2 which can be used separately from the main device 1, the user can place the separate device 2 at a location easily visible to the user, and can perform the blood pressure measurement while looking at the liquid crystal display 13 of the separate device 2. Furthermore, the user can take the separate device 2 which stores the measured data to a medical institution, and can show the measured data, such as the blood pressure value, to a doctor. In this case, as shown in FIG. 3B, the user takes the separate device 2, which is in an off-state, to the medical institution, and the user pushes the power switch 17 of the separate device 2 when needed (step S20). By this, the separate device 2 is activated (step S21), and the data stored in the memory 14 is displayed on the liquid crystal display 13 (step S22). When thirty seconds elapse after displaying the stored data (step S23), the separate device 2 is automatically turned off (step S24). Or, the separate device 2 may continue a power-on state until the power switch 17 is pushed again.

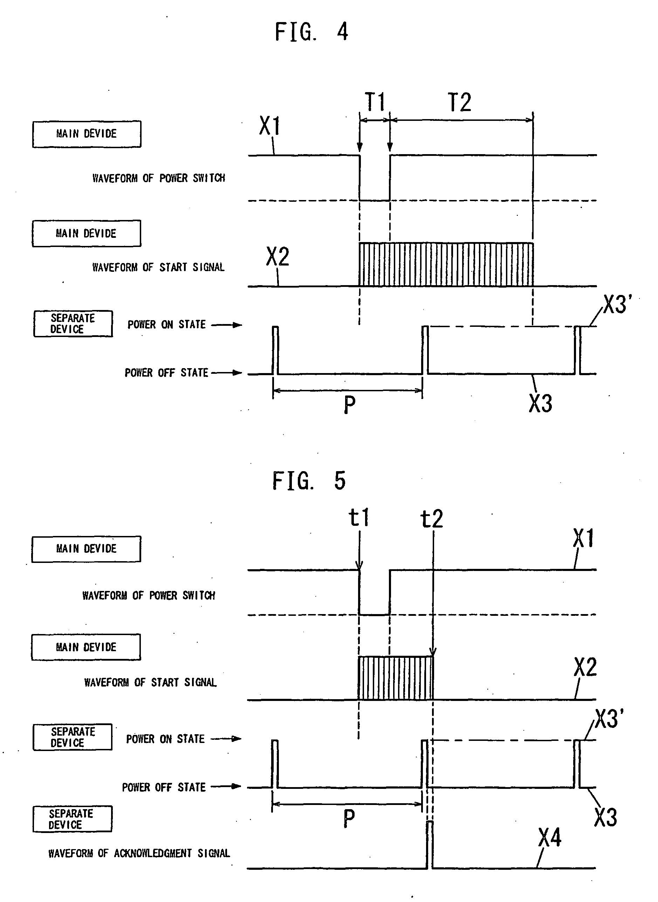

Hereinafter, the method of the wireless communication between the main device 1 and the separate device 2 performed when these devices are activated will be described in detail with reference to FIG. 4.

When the power switch 10 of the main device 1 is pushed (see a waveform X1 of FIG. 4) when the main device 1 is in a power-off state, the main device 1 is activated, and the main device 1 transmits the start signal (see a waveform X2 of FIG. 4) via the wireless communication means 11.

On the other hand, when the separate device 2 is in a power-off state, it shifts (moves) from the power-off state to a power-on state, which is an infinitesimal time period, on its own in a predetermined cycle P (see a waveform X3 of FIG. 4) to check whether the start signal is transmitted from the main device 1 or not. And, if the separate device 2 receives the start signal from the main device 1 when the separate device 2 shifts from the power-off state to the power-on state, the separate device 2 is activated (in other words, the separate device 2 continues the power-on state as shown by an alternate long and short dash line X3' of FIG. 4.). If the separate device 2 does not receive the start signal when it shifts from the power-off state to the power-on state, it returns to the power-off state again, and it shifts periodically again from the power-off state to the power-on state.

It should be noted that the separate device 2 consumes a comparatively large amount of current, e.g. about 1 milliampere, when it shifts from the power-off state to the power-on state. So, if the cycle P in which the separate device 2 shifts from the power-off state to the power-on state on its own is short, a large amount of current is consumed, whereby a remaining battery level is reduced fast. Therefore, it is preferable that the cycle P is set as long as possible.

However, for the separate device 2, in order to reliably receive the start signal transmitted from the main device 1, in other words, in order to shift from the power-off state to the power-on state at least once a period during which the start signal is transmitted, the cycle P should be shorter than a duration time of the start signal.

So, in this embodiment, as shown by the waveform X2 of FIG. 4, the main device 1 transmits the start signal continuously over a period longer than the period T1 during which the power switch 10 is operated (pushed) by the user to activate the main device 1. In more detail, the period T1 in FIG. 4 shows a period of time from when the user pushes the power switch 10 to activate the main device 1 till when the user releases the power switch 10. The main device 1 starts to transmit the start signal at the same time when the power switch 10 is pushed to activate the main device 1, and it continuously transmits the start signal over the period T1 during which the power switch 10 is operated, and also, it continuously transmits the start signal over a predetermined period T2 (for example, several hundreds milliseconds) after the completion of the operation of the power switch (in other words, after the user releases the power switch 10).

In this case, as long as the cycle P is not beyond the period T1 +T2 during which the start signal is transmitted, the separate device 2 can shift from the power-off state to the power-on state at least once the period T1 +T2 during which the start signal is transmitted and can receive the start signal transmitted from the main device 1 reliably. Therefore, it is possible to set the cycle P to an appropriate period which is not beyond the period T1+T2, whereby it is possible to constrain the power consumption of the separate device 2, as compared with a case where the start signal is transmitted only in the period T1 during which the power switch is operated. That is, it is possible to constrain the energy necessary for the wireless communication between the main device 1 and the separate device 2, whereby it is possible to reduce the consumption energy of the whole blood pressure measuring device. Furthermore, it is possible to reduce the power supply source 16 in size and weight, whereby it is possible to reduce the whole device in size and weight.

By the way, in the blood pressure measuring device of this embodiment, the main device 1 transmits the start signal, the power-off signal, and the measured data to the separate device 2. That is, the wireless communication means 11 of the main device 1 and the wireless communication means 12 of the separate device 2 have a one-way communication function, that is, a function in which only the main device 1 can transmit data to the separate device 2. Therefore, it is possible to simplify the constitution of the wireless communication means 11 and 12 and to reduce the size and the weight of the wireless communication means 11 and 12. Furthermore, because the power consumption of the wireless communication is reduced, it is possible to reduce the power supply sources 9 and 16 in size and weight.

### (Second embodiment)

The basic composition of the blood pressure measuring device of a second embodiment is identical to the first embodiment, so similar parts to the first embodiment are identified by the same reference character and no duplicate explanation is made here.

In this embodiment, the method of the wireless communication between the main device 1 and the separate device 2 performed when the main device 1 and the separate device 2 are activated is different from that of the first embodiment. The wireless communication means 11 of the main device 1 and the wireless communication means 12 of the separate device 2 of this embodiment have a two-way communication function which transmits data in both directions.

Hereinafter, the method of the wireless communication between the main device 1 and the separate device 2 of this embodiment on start-up will be described in detail with reference to FIG. 5.

As shown in FIG. 5, as is the case with the first embodiment, when the separate device 2 is in the power-off state, the separate device 2 shifts from the power-off state to the power-on state on its own in a predetermined cycle P, and checks whether the start signal is transmitted from the main device 1 or not (see the waveform X3 of FIG. 5).

When the power switch 10 of the main device 1 is pushed (see time t1 of FIG. 5), the main device 1 is activated, and the main device 1 starts to transmit the start signal (see the waveform X2 of FIG. 5).

When the separate device 2 receives the start signal when it shifts to the power-on state, it is also activated (in other words, it continues the power-on state as shown by the alternate long and short dash line X3' of FIG. 5.). And, upon activation, the separate device 2 transmits an acknowledgment signal to the main device 1 via the wireless communication means 12 (see a waveform X4 of FIG. 5).

When the main device 1 receives the acknowledgment signal (see time t2 of FIG. 5) via the wireless communication means 11, it stops transmitting the start signal. That is, the main device 1 starts to transmit the start signal at the same time when the power switch 10 is operated to activate the main device 1, and it continuously transmits the start signal until it receives the acknowledgment signal from the separate device 2.

In this case, because the start signal is continuously transmitted from the main device 1 until the main device 1 receives the acknowledgement signal from the separate device 2, it is possible to set the cycle P, in which the separate device 2 shifts from the power-off state to the power-on state on its own, long. Furthermore, because the main device 1 stops transmitting the start signal at the time when it receives the acknowledgement signal, it is possible to reduce the waste power consumption of the main device 1. Therefore, it is possible to reduce the power consumption of both of the main device 1 and the separate device 2, whereby it is possible to reduce the power supply sources 19 and 16 of both the devices in size and weight.

Or, the main device 1 may be configured to continuously transmit the start signal over a predetermined period after the completion of the operation of the power switch and stop transmitting the start signal when it receives the acknowledgment signal from the separate device 2.

### (Third embodiment)

The basic composition of the blood pressure measuring device of a third embodiment is identical to the first and second embodiments, so similar parts to these embodiments are identified by the same reference character and no duplicate explanation is made here.

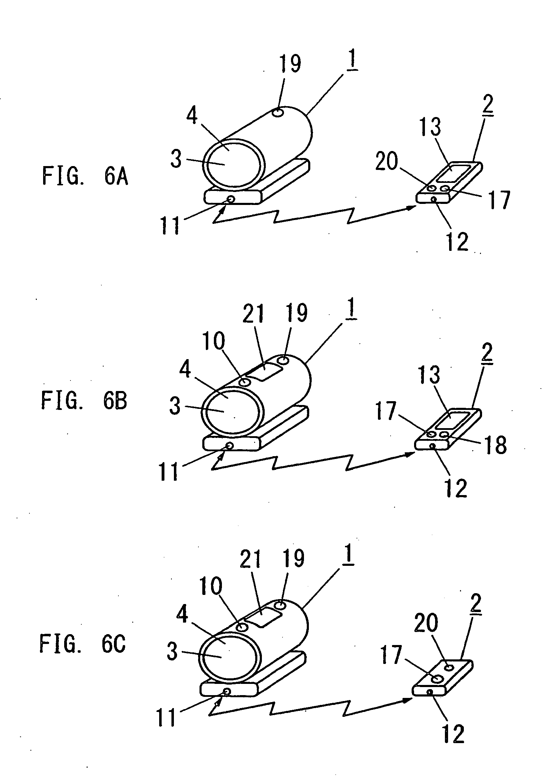

In the first embodiment, both of the main device 1 and the separate device 2 have a power switch, respectively, but in this embodiment, as shown in FIG. 6A, only the separate device 2 has a power switch 17 (that is, in this embodiment, "one device" of claim 1 is the separate device 2, and "the other device" is the main device 1.)

In this embodiment, as is the case with the second embodiment, the wireless communication means 11 of the main device 1 and the wireless communication means 12 of the separate device 2 have a two-way communication function.

When the power switch 17 of the separate device 2 is pushed when the separate device 2 is in the power-off state, the separate device 2 is activated, and the separate device 2 starts to transmit the start signal via the wireless communication means 12.

On the hand, when the main device 1 is in the power-off state, the main device 1 shifts from the power-off state to the power-on state on its own in a predetermined cycle P, and checks whether the start signal is transmitted from the separate device 2 or not. If the main device 1 receives the start signal when it shifts from the power-off state to the power-on state, the main device 1 is activated. If the main device does not receive the start signal when it shifts from the power-off state to the power-on state, it returns to the power-off state again, and it shifts periodically again from the power-off state to the power-on state.

In this embodiment, the separate device 2 transmits the start signal continuously over a period longer than the period during which the power switch 17 is operated by the user to activate the separate device 2. In more detail, the separate device 2 starts to transmit the start signal at the same time when the power switch 17 is pushed to activate the separate device 2, and it continuously transmits the start signal over the period during which the power switch 17 is pushed, and also, it continuously transmits the start signal over a predetermined period after the completion of the operation of the power switch.

Or, as is the case with the second embodiment, the main device 1 may transmit the acknowledgment signal to the separate device 2 when it received the start signal, and the separate device 2 may stop transmitting the start signal when it receives the acknowledgment signal from the main device 1.

In this embodiment, the separate device 2 has a main operation part 20 for providing instructions to the main device 1 to perform the blood pressure measurement, and it is possible for the separate device 2 to provide instructions to the main device 1 via the wireless communication means 12.

After the completion of the blood pressure measurement, the measured data is transmitted from the main device 1 to the separate device 2 via the wireless communication, and the measured data is displayed on the liquid crystal display 13 of the separate device 2.

### (Fourth embodiment)

The basic composition of the blood pressure measuring device of a fourth embodiment is identical to the first and second embodiments, so similar parts to these embodiments are identified by the same reference character and no duplicate explanation is made here.

As shown in FIG. 6B, in this embodiment, the main device 1 and the separate device 2 have the two-way communication function, and they are configured so that when either the power switch 10 of the main device 1 or the power switch 17 of the separate device 2 is operated, the other device is also activated.

In more detail, when the main device 1 and the separate device 2 are both in the power-off state, each of them shifts from the power-off state to the power-on state on its own in a predetermined cycle P in order to check the start signal.

When either the power switch 10 of the main device 1 or the power switch 17 of the separate device 2 is operated, the device having the operated power switch is activated, and then it transmits the start signal to the other device by wireless communication. When the other device receives the start signal from the one device when it shifts from the power-off state to the power-on state, the other device is also activated.

Concretely speaking, when the power switch 10 of the main device 1 is operated, the main device 1 is activated, and then it transmits the start signal to the separate device 2 by wireless communication. When the separate device 2 receives the start signal from the main device 1 when it shifts from the power-off state to the power-on state, the separate device 2 is also activated. Or, when the power switch 17 of the separate device 2 is operated, the separate device 2 is activated, and then it transmits the start signal to the main device 1 by wireless communication. When the main device 1 receives the start signal from the separate device 2 when it shifts from the power-off state to the power-on state, the main device 1 is also activated.

In this embodiment, too, the start signal is transmitted continuously over a period longer than the period during which the power switch 10 or 17 is operated by the user to activate the device. That is, the device having the operated power switch starts to transmit the start signal at the same time when the power switch is pushed, and it continuously transmits the start signal over the period during which the power switch is pushed by the user, and it also continuously transmits the start signal in a predetermined period after the operation of the power switch was finished. Or, the device which received the start signal may transmit the acknowledgment signal, and the device which is transmitting the start signal may stop transmitting the start signal when it receives the acknowledgment signal.

In addition, the main device 1 of this embodiment also has a liquid crystal display 21 for displaying various information, such as measured data. So, the user can perform the blood pressure measurement while looking at the liquid crystal display of the main device 1 and/or the separate device 2.

### (Fifth embodiment)

The basic composition of the blood pressure measuring device of a fifth embodiment is identical to the fourth embodiment, so similar parts to these embodiments are identified by the same reference character and no duplicate explanation is made here.

In the fourth embodiment, the separate device 2 has the liquid crystal display 13, but in this embodiment, as shown in FIG. 6C, only the main device 1 has the liquid crystal display 21. That is, the separate device 2 of this embodiment has the power switch 17, the main operation part 20, and the wireless communication means 12.

As is the case with the fourth embodiment, the main device 1 and the separate device 2 of this embodiment have the two-way communication function which transmits data in both directions, and they are configured so that, when either the power switch 10 of the main device 1 or the power switch 17 of the separate device 2 is operated, the other device is also activated.

Or, only either one device of the main device 1 and the separate device 2 may have a function to transmit the start signal.

### (Sixth embodiment)

The basic composition of the blood pressure measuring device of a sixth embodiment is identical to the first to fourth embodiments, so similar parts to these embodiments are identified by the same reference character and no duplicate explanation is made here.

In this embodiment, as compared with the first to fourth embodiments, only the constitution of the separate device 2 differs.

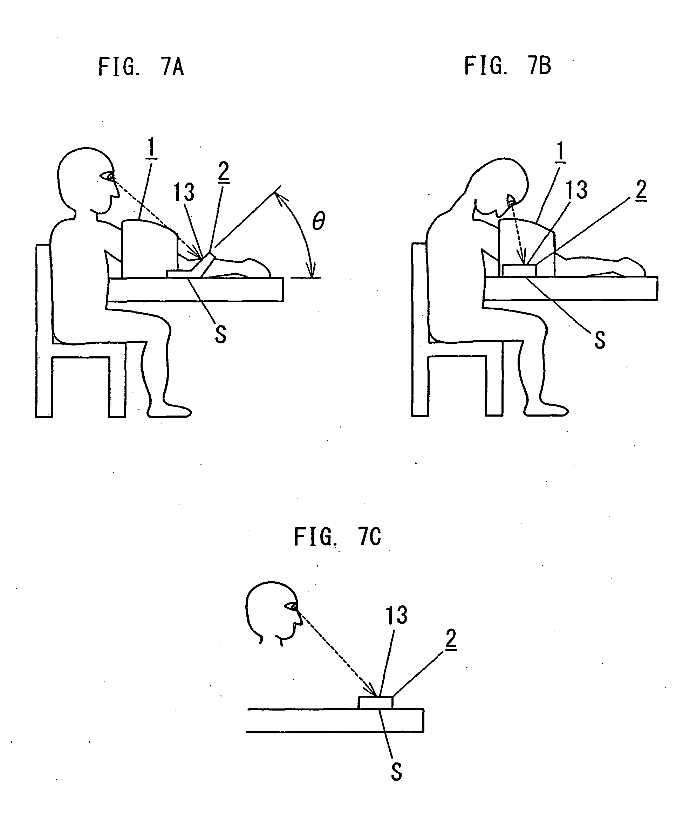

In the separate device 2 of this embodiment, as shown in FIG. 7A, the liquid crystal display 13 is inclined at a predetermined angle θ with respect to a placing surface S, such as a table. When the liquid crystal display 13 is not inclined, as shown in FIG. 7B, the user is forced to be a slouching and cramped posture when looking at the liquid crystal display 13 during blood pressure estimation, which may affects accuracy of the blood pressure measurement.

And, as shown in FIG. 7C, if the user places the separate device 2 at a distant location, he or she has to look at the liquid crystal display 13 from an oblique direction, so he or she may have trouble in seeing the displayed contents.

On the other hand, in this embodiment, because the liquid crystal display 13 is inclined at a predetermined angle θ, the user can see the liquid crystal display 13 placed at a distant location straight, so the user can perform the blood pressure measurement in a natural position, which does not affect the accuracy of the blood pressure measurement, while looking at the liquid crystal display 13.

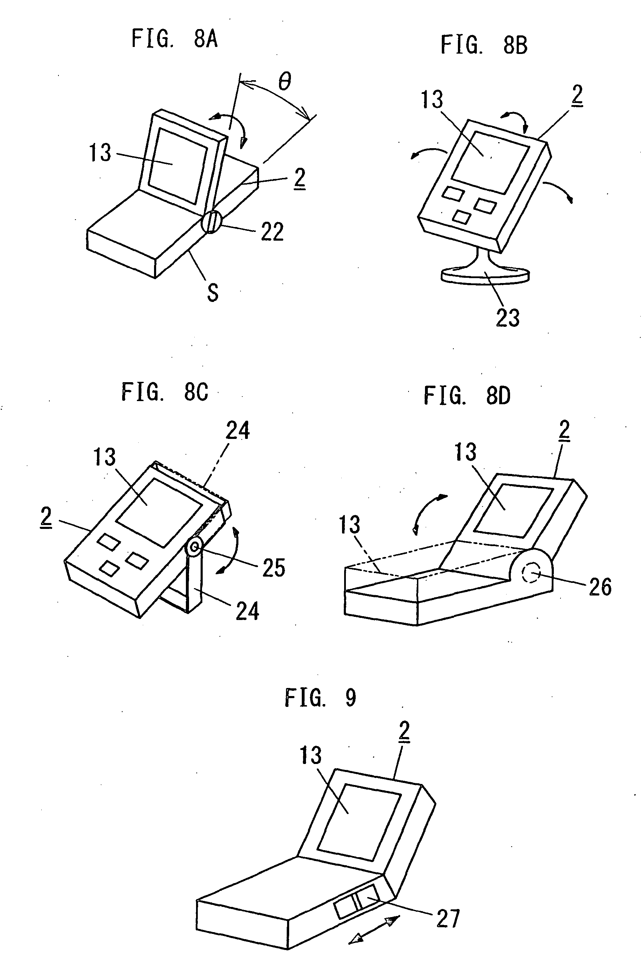

Preferably, as shown in FIG. 8A, the separate device 2 is configured so that the inclination angle θ of the liquid crystal display 13 is adjustable within a predetermined range e.g. 30 degrees to 60 degrees. In FIG. 8A, the inclination angle θ of the liquid crystal display 13 can be adjusted by operating an angular adjustment knob 22.

Or, as shown in FIG. 8B, the separate device 2 may be supported by a stand 23 rotatably in vertical and horizontal directions.

Or, as shown in FIG. 8C, the separate device 2 having a flat shape may have a U-shaped stand 24. The U-shaped stand 24 is attached to both side surfaces of the separate device 2, and is rotatable about an axis 25. By setting the stand 24, it is possible to tilt the liquid crystal display 13 with respect to the placing surface. And when the separate device 2 is not used, it is possible to retract the stand 24 by rotating the stand 24 until it overlaps the side surfaces of the separate device 2 (see an alternate long and short dash line of FIG. 8C), so that the separate device 2 becomes the flat shape . So, it is convenient to carry the separate device 2.

Or, as shown in FIG. 8D, the separate device 2 may be formed foldably. The liquid crystal display 13 is formed in an upper half of the separate device 2, and it is rotatable about an axis 26 with respect to a lower half of the separate device 2. When the separate device 2 is not used, the separate device 2 can be folded in the middle (see an alternate long and short dash line of FIG. 8D). In this case, too, it is convenient to carry the separate device 2. Furthermore, it is possible to adjust the inclination angle θ of the liquid crystal display 13 freely.

Or, as shown in FIG. 9, a view angle of the liquid crystal display 13 may be adjusted by a sliding lever 27. When the sliding lever 27 is slid in one direction e.g. to the back side, the view angle is ascended, and when the sliding lever 27 is slid in the other direction, e.g. to the front side, the view angle is descended. In this case, it is not necessary to provide a mechanism for rotating the liquid crystal display 13, so it is possible to increase the durability.

## Claims

1. A blood pressure measuring device comprising:
a main device (1): said main device having a pressurizing means (5) for inflating a cuff (3), a control means (8) for controlling said pressurizing means to measure a blood pressure of a user, and a wireless communication means (11);
a separate device (2) capable of being used separately from said main device:
said separate device having a wireless communication means (12) for performing a wireless communication with said wireless communication means (11) of said main device and at least one of a display means (13) for displaying data received from said main device via the wireless communication and an operation means (18) for operating said main device via the wireless communication,
wherein
at least one device of said main device and said separate device has a power switch (10,17),
one device having said power switch is configured to be activated when said power switch is operated when it is in a power-off state, and then to transmit a start signal to the other device by the wireless communication
the other device is configured to shift periodically from a power-off state to a power-on state on its own in a predetermined cycle (P) when it is in the power-off state in order to check said start signal, and to be activated if it receives said start signal when it shifts from the power-off state to the power-on state, and to return to the power-off state if it does not receive said start signal when it shifts from the power-off state to the power-on state,
wherein said one device is configured to start transmitting said start signal at the same time when said power switch is operated to activate the device, and the predetermined cycle (P) in which the other device shifts from the power-off state to the power-on state on its own is not beyond the period during which the start signal is transmitted,
**characterised in that**
said one device is configured to transmit said start signal continuously over a period longer than a period during which said power switch is operated to activate said one device, wherein said one device is configured to continuously
transmit said start signal over a period (T₁) during which said power switch is operated, and to continuously transmit said start signal over a predetermined period (T₂) after completion of the operation of said power switch.

2. A blood pressure measuring device comprising:
a main device (1): said main device having a pressurizing means (5) for inflating a cuff (3), a control means (8) for controlling said pressurizing means (5) to measure a blood pressure of a user, and a wireless communication means (11);
a separate device (2) capable of being used separately from said main device:
said separate device having a wireless communication means (12) for performing a wireless communication with said wireless communication means (11) of said main device (1) and at least one of a display means (13) for displaying data received from said main device via the wireless communication and an operation means (18) for operating said main device via the wireless communication,
wherein
at least one device of said main device and said separate device has a power switch (10,17),
one device having said power switch is configured to be activated when said power switch is operated when it is in a power-off state, and then to transmit a start signal to the other device by the wireless communication,
the other device is configured to shift periodically from a power off state to a power-on state on its own when it is in the power-off state in order to check said start signal, and to be activated if it receives said start signal when it shifts from the power-off state to the power on state, and to return to the power-off state if it does not receive said start signal when it shifts from the power-off state to the power-on state,
wherein said one device is configured to start to transmit said start signal at the same time when said power switch is operated to activate the device,
**characterised in that**
said the other device is configured to transmit an acknowledgment signal when it receives the start signal, and said one device is configured to stop transmitting said start signal when it receives the acknowledgment signal from the other device.

## Patentansprüche

1. Ein Blutdruckmessgerät umfassend:
eine Haupteinheit (1): die Haupteinheit umfasst eine Druckerzeugungseinrichtung (5) zum Aufpumpen einer Manschette (3), eine Kontrolleinheit (8) zum Steuern der Druckerzeugungseinrichtung, um einen Blutdruck eines Nutzers zu messen, und eine drahtlose Kommunikationseinrichtung (11);
eine separate Einheit (2), die in der Lage ist, separat von der Haupteinheit genutzt zu werden: die separate Einheit umfasst eine drahtlose Kommunikationseinrichtung (12) zum Durchführen einer drahtlosen Kommunikation mit der drahtlosen Kommunikationseinrichtung (11) der Haupteinheit und zumindest eine Anzeigeeinrichtung (13) zum Anzeigen von Daten, die von der Haupteinheit über die drahtlose Kommunikation erhalten werden, und eine Bedieneinrichtung (18) zum Bedienen der Haupteinheit über die drahtloses Kommunikation,
wobei zumindest ein Gerät, das Hauptgerät oder das separate Gerät, einen Netzschalter (10, 17) umfasst,
ein Gerät, das den Netzschalter aufweist, ist konfiguriert, um aktiviert zu werden, wenn der Netzschalter in einem ausgeschalteten Zustand bedient wird, und um dann ein Startsignal an das andere Gerät über die drahtlose Kommunikation auszusenden,
das andere Gerät ist konfiguriert, um periodisch von einem ausgeschalteten Zustand zu einem eingeschalteten Zustand selbständig in einem vorbestimmten Zyklus (P) zu wechseln, wenn es sich in dem ausgeschalteten Zustand befindet, um das Startsignal zu überprüfen und um aktiviert zu werden, falls es das Startsignal erhält, wenn es von dem ausgeschalteten Zustand in den eingeschalteten Zustand wechselt, und um in den ausgeschalteten Zustand zurückzukehren, falls es das Startsignal nicht erhält, wenn es von dem ausgeschalteten Zustand in den eingeschalteten Zustand wechselt,
wobei das eine Gerät konfiguriert ist, um mit der Übertragung des Startsignals zur gleichen Zeit zu beginnen, wenn der Netzschalter bedient wird, um das Gerät zu aktivieren, und der vorbestimmte Zyklus (P), in welchem das andere Gerät von dem ausgeschalteten Zustand in den eingeschalteten Zustand selbständig wechselt, ist nicht größer als die Zeitdauer, in welcher das Startsignal übertragen wird,
**dadurch gekennzeichnet, dass**
das eine Gerät konfiguriert ist, um das Startsignal kontinuierlich über eine Periodenlänge zu übertragen, die länger ist als eine Periode, während der der Netzschalter bedient wird, um das Gerät anzuschalten, wobei das eine Gerät konfiguriert ist, um kontinuierlich das Startsignal über eine Periode (T₁) zu übertragen, während der der Netzschalter bedient wird, und um kontinuierlich das Startsignal über eine vorbestimmte Periode (T₂) zu übertragen, nachdem die Bedienung des Netzschalters beendet ist.

2. Ein Blutdruckmessgerät umfassend:
eine Haupteinheit (1): die Haupteinheit umfasst eine Druckerzeugungseinrichtung (5) zum Aufpumpen einer Manschette (3), ein Kontrolleinheit (8) zum Kontrollieren der Druckerzeugungseinrichtung (5), um einen Blutdruck eines Nutzers zu messen, und eine drahtlose Kommunikationseinrichtung (11);
ein separates Gerät (2), das in der Lage ist, separat von dem Hauptgerät genutzt zu werden: das separate Gerät umfasst eine drahtlose Kommunikationseinrichtung (12) zum Durchführen einer drahtlosen Kommunikation mit der drahtlosen Kommunikationseinrichtung (11) der Haupteinheit (1) und zumindest eine Anzeigeeinrichtung (13) zum Anzeigen von Daten, die von dem Haupteinheit über die drahtlose Kommunikation erhalten werden, und eine Bedieneinrichtung (18) zum Bedienen des Hauptgerätes über die drahtloses Kommunikation,
wobei
zumindest eines der Geräte, das aus dem Hauptgerät und dem separaten Gerät ausgewählt wird, einen Netzschalter (10, 17) aufweist,
ein Gerät, das den Netzschalter aufweist, konfiguriert ist, um aktiviert zu werden, wenn der Netzschalter in einem ausgeschalteten Zustand bedient wird, und um dann ein Startsignal an das andere Gerät über die drahtlose Kommunikation zu übertragen,
das andere Gerät konfiguriert ist, um periodisch zwischen einem ausgeschalteten Zustand und einem eingeschalteten Zustand selbständig zu wechseln, wenn es sich in dem ausgeschalteten Zustand befindet, um das Startsignal zu überprüfen und um aktiviert zu werden, falls es das Startsignal empfängt, wenn es von dem ausgeschalteten Zustand in den eingeschalteten Zustand wechselt, und um in den ausgeschalteten Zustand zurückzukehren, falls es das Startsignal nicht empfängt, wenn es von dem ausgeschalteten Zustand in den eingeschalteten Zustand wechselt,
wobei das eine Gerät konfiguriert ist, um mit der Übertragung des Startsignals zur gleichen Zeit zu beginnen, wenn der Netzschalter bedient wird, um das Gerät zu aktivieren,
**dadurch gekennzeichnet, dass**
das andere Gerät konfiguriert ist, um ein Bestätigungssignal zu übertragen, wenn es das Startsignal erhält, und das eine Gerät konfiguriert ist, um die Übertragung des Startsignals anzuhalten, wenn es das Bestätigungssignal von dem anderen Gerät empfängt.

## Revendications

1. Dispositif de mesure de pression artérielle comprenant :
un dispositif principal (1) ; ledit dispositif principal comportant des moyens de mise sous pression (5) pour gonfler un ballonnet (3), des moyens de commande (8) pour commander lesdits moyens de mise sous pression pour mesurer une pression artérielle d'un utilisateur, et des moyens de communication sans fil (11) ;
un dispositif séparé (2) qui peut être utilisé séparément dudit dispositif principal ;
ledit dispositif séparé comportant des moyens de communication sans fil (12) pour effectuer une communication sans fil avec lesdits moyens de communication sans fil (11) dudit dispositif principal et au moins l'un de moyens d'affichage (13) pour afficher des données reçues dudit dispositif principal par l'intermédiaire de la communication sans fil et de moyens de mise en oeuvre (18) pour mettre en oeuvre ledit dispositif principal par l'intermédiaire de la communication sans fil,
dans lequel
au moins un dispositif dudit dispositif principal et dudit dispositif séparé comporte un commutateur d'alimentation (10, 17),
un dispositif comportant ledit commutateur d'alimentation est configuré pour s'activer lorsque ledit commutateur d'alimentation est actionné alors qu'il est dans un état hors tension, et pour transmettre ensuite un signal de démarrage à l'autre dispositif par la communication sans fil,
l'autre dispositif est configuré pour passer périodiquement d'un état hors tension dans un état sous tension de lui-même dans un cycle prédéterminé (P) lorsqu'il est dans l'état hors tension afin de contrôler ledit signal de démarrage, et pour être activé s'il reçoit ledit signal de démarrage lorsqu'il passe de l'état hors tension dans l'état sous tension, et pour retourner dans l'état hors tension s'il ne reçoit pas ledit signal de démarrage lorsqu'il passe de l'état hors tension dans l'état sous tension,
dans lequel ledit un dispositif est configuré pour débuter la transmission dudit signal de démarrage à l'instant auquel ledit commutateur d'alimentation est actionné pour activer le dispositif et quand le cycle prédéterminé (P) dans lequel l'autre dispositif passe de l'état hors tension dans l'état sous tension de lui-même n'est pas en-dehors de la période pendant laquelle le signal de démarrage est transmis,
**caractérisé en ce que**
ledit un dispositif est configuré pour transmettre ledit signal de démarrage continuellement sur une période plus longue qu'une période pendant laquelle ledit commutateur d'alimentation est actionné pour activer ledit un dispositif,
dans lequel ledit un dispositif est configuré pour transmettre continuellement ledit signal de démarrage sur une période (T₁) pendant laquelle ledit commutateur d'alimentation est actionné, et pour transmettre continuellement ledit signal de démarrage sur une période prédéterminée (T₂) à la fin de l'actionnement dudit commutateur d'alimentation.

2. Dispositif de mesure de pression artérielle comprenant :
un dispositif principal (1) ; ledit dispositif principal comportant des moyens de mise sous pression (5) pour gonfler un ballonnet (3), des moyens de commande (8) pour commander lesdits moyens de mise sous pression (5) pour mesurer une pression artérielle d'un utilisateur, et des moyens de communication sans fil (11) ;
un dispositif séparé (2) qui peut être utilisé séparément dudit dispositif principal ;
ledit dispositif séparé comportant des moyens de communication sans fil (12) pour effectuer une communication sans fil avec lesdits moyens de communication sans fil (11) dudit dispositif principal (1) et au moins l'un de moyens d'affichage (13) pour afficher des données reçues dudit dispositif principal par l'intermédiaire de la communication sans fil et de moyens de mise en oeuvre (18) pour mettre en oeuvre ledit dispositif principal par l'intermédiaire de la communication sans fil,
dans lequel
au moins un dispositif dudit dispositif principal et dudit dispositif séparé comporte un commutateur d'alimentation (10, 17),
un dispositif comportant ledit commutateur d'alimentation est configuré pour s'activer lorsque ledit commutateur d'alimentation est actionné alors qu'il est dans un état hors tension, et pour transmettre ensuite un signal de démarrage à l'autre dispositif par la communication sans fil,
l'autre dispositif est configuré pour passer périodiquement d'un état hors tension dans un état sous tension de lui-même lorsqu'il est dans l'état hors tension afin de contrôler ledit signal de démarrage, et pour être activé s'il reçoit ledit signal de démarrage lorsqu'il passe de l'état hors tension dans l'état sous tension, et pour retourner dans l'état hors tension s'il ne reçoit pas ledit signal de démarrage lorsqu'il passe de l'état hors tension dans l'état sous tension,
dans lequel
ledit un dispositif est configuré pour débuter la transmission dudit signal de démarrage à l'instant auquel ledit commutateur d'alimentation est actionné pour activer le dispositif,
**caractérisé en ce que**
ledit autre dispositif est configuré pour transmettre un signal d'acquittement lorsqu'il reçoit le signal de démarrage, et ledit un dispositif est configuré pour arrêter la transmission dudit signal de démarrage lorsqu'il reçoit le signal d'acquittement de l'autre dispositif.
